# EUROPEAN PATENT APPLICATION

(11) **EP 4 534 648 A1**
(43) Date of publication of application: **09.04.2025**
(21) Application number: 23811174.4
(22) Date of filing: 26.05.2023
(51) Int. Cl.: C12N 1/21, C12P 5/00

(54) **CO-CULTURE SYSTEM AND METHOD FOR SYNTHESIZING TERPENE**

(30) Priority: 26.05.2022 CN 202210590501
(71) Applicant: Oxford University (Suzhou) Science and Technology CO. LTD, Suzhou, Jiangsu 215123 (CN)
(72) Inventor: PANDREKA, Avinash, Suzhou, Jiangsu 215123 (CN); HUANG, Lule, Suzhou, Jiangsu 215123 (CN); CAO, Yang, Suzhou, Jiangsu 215123 (CN)
(74) Representative: Torggler & Hofmann Patentanwälte - Innsbruck
(86) International application number: PCT/CN2023/096509
(87) International publication number: WO 2023/227104

(57) **Abstract**

The present invention discloses a co-cultivation system and method for synthesizing terpenes, the system comprising at least one feeding strain and at least one production strain, wherein the feeding strain is used to provide mevalonic acid and the production strain is used to synthesize terpenes from the mevalonic acid. The co-cultivation system and method of the present invention can be applied to the production of any terpenes and can increase the total yield and the conversion rate of terpenes.

## Description

### Technical field

The present invention relates to the field of microorganism, and in particular provides a co-cultivation system and method for synthesizing terpenes.

### Background

Terpenoids are widely used in different industrial applications such as flavors, fragrances, biofuels, pharmaceuticals, rubber, and pesticides. These compounds are produced by plants, but often in low levels that are not enough to meet industrial needs.

Terpenes and their oxygen-containing derivatives are derived from mevalonic acid or deoxy-D-xylulose 5-phosphate, with molecular skeletons constructed from isoprene units (C₅ unit) as the basic structural unit. The oxygen-containing derivatives can be alcohols, aldehydes, ketones, carboxylic acids, esters, *etc.* Terpenes are a common type of terpenoids. Most of these molecules are complex, and chemical processes for their synthesis involve many steps and complex chemical reactions, resulting in low yield, incorrect stereochemistry, and high cost. In recent years, synthetic biology has become a rapidly expanding and advancing field of research, with widespread applications in sustainable and greener synthesis. A typical example of terpenoid production by synthetic biology is the synthesis of artemisinin via artemisinic acid which is produced by the biosynthesis of the sesquiterpene precursor amorpha-4,11-diene followed by its sequential oxidation to the alcohol, aldehyde and then the acid. However, this complex polygenic system is subject to metabolic stress and pathway constraints, such as metabolic fluxes and intermediates that are released into the medium and do not re-enter through the cell wall.

In one process for pinene biosynthesis, two strains are grown in medium and the cells harvested then transferred to phosphate buffered saline to produce terpenes. In one sabinene biosynthesis process, bacteria must be grown to produce mevalonic acid and then the mevalonic acid-containing medium is used with the growth medium of sabinene biosynthetic strains. These processes are complex and require stringent process control while the use of isoprene units as exchange metabolites may also stress the bacteria. Most of the research in terpene metabolic engineering are focused on engineering the microbial genome, evolving isoprenoid auxotrophic mutants for enhanced terpenoid biosynthesis and metabolic intermediates which are toxic and difficult to exchange between microbes.

To date, very few processes can meet the terpenoid production levels required to support industrial needs.

### SUMMARY OF THE INVENTION

To overcome deficiencies in the prior art, the present invention develops a simple co-cultivation system for enhanced terpene biosynthesis by dividing the isoprenoid MVA pathway between microbial strains without altering their native isoprene pathways. Furthermore, use of polar molecules as transfer metabolites between strains prevents partitioning into organic solvents. The toxic hydrophobic product can be extracted from the microbial culture with an organic solvent. Therefore, the present invention provides a co-cultivation system, which comprises a feeding strain for synthesizing mevalonic acid as an exchange metabolite and a production strain for synthesizing terpenes using mevalonic acid as the raw material. The co-cultivation system provides an adjustable solution for heterologous protein expression. The co-cultivation system provided by the present invention reduces the metabolic stress of microorganisms by separating the synthesis of metabolite intermediates between microorganisms, thereby improving the conversion rate of intermediates and the yield of target compounds.

A first aspect of the present invention provides a co-cultivation system for synthesizing terpenes, comprising at least one feeding strain and at least one production strain, wherein the feeding strain is used to provide mevalonic acid while the production strain is used to synthesize terpenes from the mevalonic acid.

According to some embodiments of the present invention, the feeding strain contains a first plasmid, the first plasmid contains a gene for synthesizing mevalonic acid and does not contain a prenylpyrophosphate synthase gene. According to some embodiments of the present invention, the feeding strain further contains a second plasmid, the second plasmid is a blank plasmid without gene insertion. According to some specific embodiments of the present invention, the second plasmid is the pUC-19 plasmid without any gene insertion. According to some embodiments of the present invention, the second plasmid facilitates compensation of antibiotics with the production strain. In the present invention, the feeding strain provides mevalonic acid. The mevalonic acid is released into the medium and then enters through the cell wall of the production strain which expresses mevalonic acid genes, such as the cell membrane of the production strain containing prenyl pyrophosphate and terpene synthase.

According to some embodiments of the present invention, the production strain contains a third plasmid and a fourth plasmid. The third plasmid contains a gene for synthesizing farnesyl pyrophosphate and does not contain genes for mevalonic acid synthesis. The fourth plasmid contains either the gene of the terpene synthase or fused terpene synthase with geranylgeranyl pyrophosphate synthase.

According to some embodiments of the invention, the MVA pathway plasmid pBbA5c-MevT(CO)-T1-MBIS(CO,ispA) (pMVA) from Addgene was modified to obtain expression modules for the feeding strain and production strain. The pMVA plasmid is a medium copy number plasmid containing two modules, one for the production of mevalonic acid and the other for the production of farnesyl pyrophosphate (FPP). New plasmids pMevt and pMBIS were generated by cloning each module from pMVA; pMevt is a medium copy number plasmid containing the mevalonic acid production module; pMBIS is another medium copy number plasmid containing the FPP production module. pTRC-X was generated by cloning the codon-optimized terpene X synthase gene into pTRC-HisA. In the present invention, feeding strains are produced by transforming the pMevt and pUC-19 plasmids into microorganisms such as *Escherichia coli (E. coli),* and production strains are produced by transforming the pMBIS and pTRC-X plasmids into microorganisms such as *E. coli.*

According to some embodiments of the present invention, the ratio of the feeding strain to the production strain is (0.25-99.75) : (99.75-0.25), such as (0.25-99.5) : (99.75-0.5), preferably (1.25-75) : (98.75-25). According to some preferred embodiments of the present invention, the ratio of the feeding strain to the production strain is (0.5-75) : (99.5-25), such as (12.5-75) : (87.5-25). In the present invention, the range of co-cultivated strains is further improved by changing the plasmids, inoculation ratio, heterologous enzyme expression, efficiency and genome modification, thereby further improving the yield of terpenes.

According to some embodiments of the present invention, the terpene includes one or more of taxadiene, α-bulnesene, valencene, α-guaiene, β-patchoulene, or aristolochene.

According to some embodiments of the present invention, the feeding strain and/or the production strain are selected from one or more of bacteria, fungi or yeasts. According to some specific embodiments of the present invention, the feeding strain and/or the production strain is *E. coli.*

According to some embodiments of the present invention, the metabolic pathway of the feeding strain and/or the production strain comprise the endogenous isoprenoid/terpene biosynthetic pathway. For example, bacteria have an endogenous MEP pathway and yeast has an endogenous MVA pathway. According to some embodiments of the present invention, the metabolic pathway of the feeding strain and/or the production strain further comprises part of the engineered MVA pathway up to prenyl pyrophosphate (GPP, FPP, GGPP, *etc.)* and terpene synthases.

In the present invention, this co-cultivation system works by dividing the many steps in the terpene biosynthetic pathway into two modules where one module is carried out in one strain and the remaining steps are carried out in the other strain. The steps of the two strains are linked by freely diffusing exchange metabolites (mevalonic acid). The metabolic stress of performing all steps in a single strain is divided between the two strains, reducing the metabolic stress on each strain and thus increasing the yield of the target product. The use of polar molecules (such as mevalonic acid) as the transfer metabolite between the two strains prevents partitioning into extractants such as decane. The two strains interact to balance the production and consumption of mevalonic acid to promote the survival and growth of both strains.

A second aspect of the present invention provides a method for synthesizing terpenes using the co-cultivation system described in the first aspect, comprising incubating the feeding strain and the production strain in the presence of an inducer and an organic phase or solid phase resin for separating the terpenes from the aqueous phase.

According to some embodiments of the present invention, an organic phase such as decane or a solid phase resin such as Diaion HP20 is used to extract the terpenes from the aqueous phase. According to the present invention, the organic phase is not limited to decane, but can be further selected from undecane and dodecane; or selected from isopropyl caprate, isopropyl laurate, isopropyl octanoate, isopropyl myristate, *etc.* According to some embodiments of the present invention, the organic phase accounts for 5% to 30% v/v of the culture, preferably 10% to 20%. The solid phase resin is not limited to Diaion HP20 but can be further selected from Dowax, HP-20, XAD7HP or HP-2MG.

According to some embodiments of the present invention, based on the total inoculation volume of the feeding strain and the production strain, the content of the feeding strain is 0.25% to 99%. According to a preferred embodiment of the present invention, based on the total inoculation volume of the feeding strain and the production strain, the content of the feeding strain is 0.25% to 99%, preferably 0.5% to 75%, such as 12.5% to 75%. According to some embodiments of the present invention, based on the total inoculation volume of the feeding strain and the production strain, the content of the feeding strain is 0.5%, 1%, 2%, 5%, 10%, 12.5%, 25%, 37.5%, 50%, 62.5%, 75%, and any value in between.

According to some embodiments of the present invention, when the terpene is taxadiene, based on the total inoculation volume of the feeding strain and the production strain, the content of the feeding strain is 0.25% to 99%, preferably 0.25% to 37.5%, such as 0.25% to 12.5% or 12.5% to 37.5%, preferably 0.25% to 12.5%, in particular preferably 0.25% to 5%, especially 0.5% to 5%.

According to some embodiments of the present invention, when the terpene is β-patchoulene, based on the total inoculation volume of the feeding strain and the production strain, the content of the feeding strain is 12.5%-75%, preferably 25%-75%, especially 37.5%-62.5%.

According to some embodiments of the present invention, when the terpene is aristolochene, based on the total inoculation volume of the feeding strain and the production strain, the content of the feeding strain is 12.5%-75%.

According to some embodiments of the present invention, the separation and purification is performed by extraction with an organic solvent, preferably the organic solvent includes decane.

The third aspect of the present invention provides the application of the co-cultivation system according to the first aspect or the method according to the second aspect in synthesizing terpenes.

A fourth aspect of the present invention provides the use of mevalonic acid as an exchange metabolite in microbial co-cultivation to synthesize terpenes.

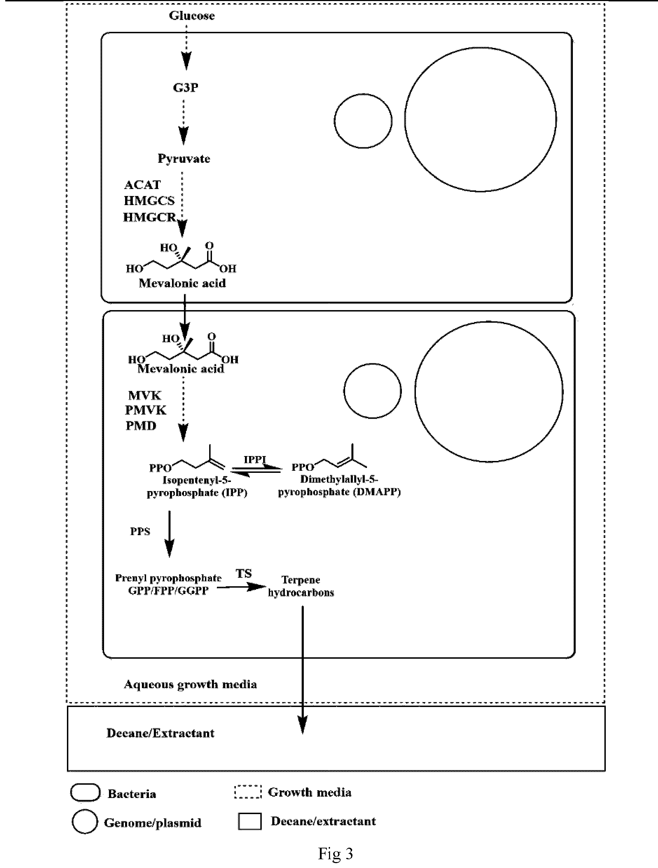

Beneficial effects of the present invention:
1. The co-cultivation system of the present invention can improve the total terpene output. Production is currently carried out in shake flasks and can yield approximately 1 g/L of terpenes, which will increase significantly when the production system is transferred to a fermenter.
2. By expressing the corresponding terpene synthase, the co-cultivation system and the method of the present invention can be applied to the production of any terpene, including but not limited to (a) production for biofuels and other applications of monoterpenes (linalool, geraniol, and 2,6-dimethyloctane) and other sesquiterpenes (bisabolene, pentene, and isocene); (b) terpene pathways in co-cultures leading to increased expression of diterpene synthase and higher yields of diterpenes which have a wide range of biological activities, including pharmaceutical effects; (c) feeding strains can be further modified to express hydrolase enzymes to degrade cellulose, lignin, polymers such as hemicellulose; (d) multiple feeding strains can be added to exploit complex materials such as agricultural and food waste; (e) these multi-strain principles can be applied to any other pathway to enhance microbial community interactions, fluxes and increase product formation; (f) different microorganisms such as yeasts, fungi and bacteria can be used to create multi-strain co-cultures or consortia.
3. Another key advantage of the present invention is that one strain (the feeding strain) provides mevalonic acid, and the mevalonic acid is released into the medium and then passes through the cell wall of the other strain (production strain) that expresses genes that use mevalonic acid, i.e., prenyl pyrophosphate synthases (Figure 3). By dividing the process into these steps, the metabolic stress of expressing heterologous enzymes on each organism is reduced, resulting in faster growth, increased strain viability, and higher volumetric yields. The co-cultivation system reduces fermentation time, and the ratio of feeding strains and production strains can be adjusted, e.g., by controlling the inoculum size.

### Description of drawings

Figure 1 shows examples of terpenes synthesized by terpene synthases from farnesyl pyrophosphate (FPP) and geranylgeranyl pyrophosphate (GGPP).
Figure 2 shows some of the prior art for cultivation systems using more than one strain for terpene production: (A) a two-strain system for pinene production using phosphate buffer, (B) a two-step cultivation process where the initial strain was grown to produce mevalonic acid, and the culture media with mevalonic acid is fed to the second strain for terpene production.
Figure 3 shows a minimal or simple two-strain co-cultivation system according to the present invention.
Figure 4 shows taxadiene biosynthesis in Example 1.1 using a single-strain cultivation system and a two-strain co-cultivation system.
Figure 5 shows the gas chromatographic analysis of taxadiene biosynthesis in Example 1.1.
Figure 6 shows taxadiene biosynthesis in Example 1.2 using a single-strain cultivation system and a two-strain co-cultivation system.
Figure 7 shows the gas chromatographic analysis of taxadiene biosynthesis in Example 1.2.
Figure 8 shows α-bulnesene biosynthesis in Example 2 using a single-strain cultivation system and a two-strain co-cultivation system.
Figure 9 shows the gas chromatographic analysis of α-bulnesene synthesis in Example 2.
Figure 10 shows valencene biosynthesis in Example 3 using a single-strain cultivation system and a two-strain co-cultivation system.
Figure 11 shows the gas chromatographic analysis of valencene biosynthesis in Example 3.
Figure 12 shows the biosynthesis of β-patchoulene in Example 4 using a single-strain cultivation system and a two-strain co-cultivation system.
Figure 13 shows the gas chromatographic analysis of β-patchoulene biosynthesis in Example 4.
Figure 14 shows aristolochene biosynthesis in Example 5 using a single-strain cultivation system and a two-strain co-cultivation system.

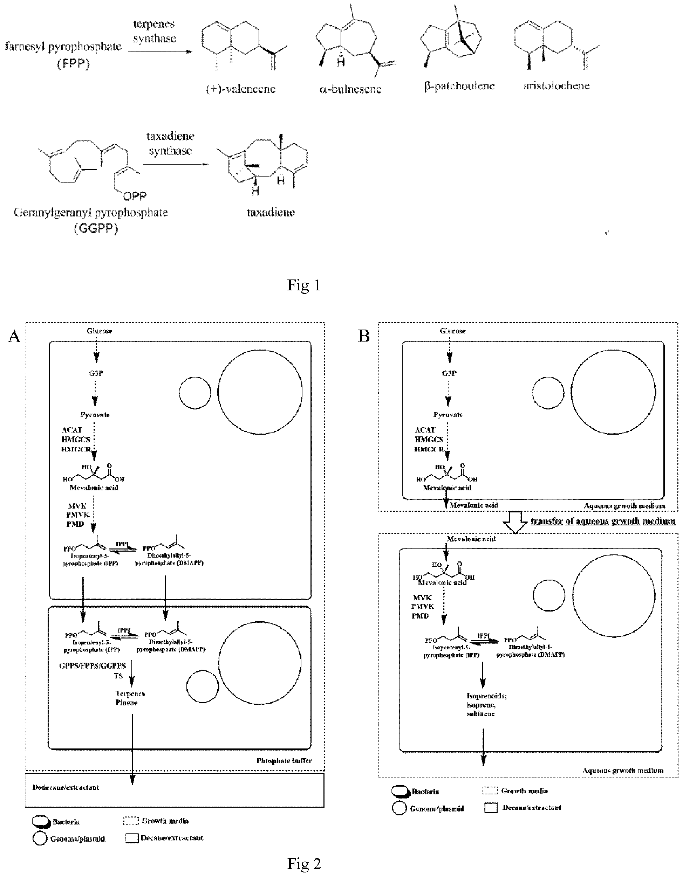

Figure 2-3 G3P: glyceraldehyde 3-phosphate; ACAT: acetyl-CoA C-acetyltransferase; HMGCS: 3-hydroxy-3-methylglutaryl-CoA synthase; HMGCR: 3-hydroxy-3-methylglutaryl-CoA reductase; MVK: mevalonic acid-5-kinase; PMVK: phosphomevalonic acid kinase; PMD: mevalonic acid pyrophosphate decarboxylase; IPPI: isopentenyl pyrophosphate isomerase; GPPS: geranyl pyrophosphate synthase; FPPS: farnesyl pyrophosphate synthase; GGPPS: geranylgeranyl pyrophosphate synthase; GPP: geranyl pyrophosphate; FPP: farnesyl pyrophosphate; GGPP: geranylgeranyl pyrophosphate; TS: terpene synthase.

### EMBODIMENT

The present application will be further described below in conjunction with specific embodiments. It should be understood that these specific embodiments are only used to illustrate the present application and not to limit the scope of the present application.

For the sake of brevity, only some numerical ranges are specifically disclosed herein. However, any lower limit can be combined with any upper limit to form an unspecified range; and any lower limit can be combined with any other lower limit to form an unspecified range, and likewise any upper limit can be combined with any other upper limit to form an unspecified range. Furthermore, each individually disclosed point or single value may itself serve as a lower or upper limit in combination with any other point or single value or with other lower or upper limits to form a range that is not expressly recited.

Unless otherwise specified, terms used in this application have their commonly known meanings as commonly understood by those skilled in the art. Unless otherwise specified, the numerical values of the parameters mentioned in this application can be measured by various measurement methods commonly used in the art (for example, can be tested according to the methods given in the examples of this application).

A list of items to which the terms "at least one of," or other similar terms are linked can mean any combination of the listed items. For example, if items A and B are listed, the phrase "at least one of A and B" means A only; B only; or A and B. In another example, if items A, B, and C are listed, the phrase "at least one of A, B, and C" means A only; or B only; C only; A and B (excluding C); A and C (excluding B); B and C (excluding A); or all of A, B, and C. Item A may contain a single component or multiple components. Item B may contain a single component or multiple components. Item C may contain a single component or multiple components.

The present application discloses a co-cultivation system comprising a feeding strain providing mevalonic acid as an exchange metabolite and a production strain using mevalonic acid as a raw material. The synthesis of metabolic intermediates followed by exchange of such intermediates between microorganisms reduces the metabolic stress of microorganisms, thereby increasing the conversion rate of intermediates and the yield of target compounds. The following numbered aspects describe various embodiments of the invention.

Embodiment 1. A co-cultivation system for synthesizing terpenes, comprising at least one feeding strain and at least one production strain, wherein the feeding strain provide mevalonic acid and the production strain synthesizes terpenes using the mevalonic acid as starting material.

Embodiment 2. The co-cultivation system according to Embodiment 1 is characterized by the feeding strain containing a first plasmid, where the first plasmid contains a gene for synthesizing mevalonic acid and does not contain a gene for synthesizing prenyl pyrophosphate, preferably, the feeding strain further contains a second plasmid, the second plasmid is a blank plasmid without gene insertion.

Embodiment 3. The co-cultivation system according to Embodiment 1 or 2, wherein the production strain contains a third plasmid and a fourth plasmid, and the third plasmid contains a gene for synthesizing farnesyl pyrophosphate and does not contain the gene for synthesizing mevalonic acid, and the fourth plasmid contains the gene for the terpene synthase or fused gene of terpene synthase with geranylgeranyl pyrophosphate synthase.

Embodiment 4. The co-cultivation system according to any one of Embodiments 1-3, wherein the ratio of the feeding strain to the production strain (0.25-99.75) : (99.75-0.25), preferably (1.25-75) : (98.75-25).

Embodiment 5. The co-cultivation system according to any one of Embodiments 1 to 4, wherein the terpene is selected from the group of taxadiene, α-bulnesene, valencene, α-guaiene, β-patchoulene or aristolochene.

Embodiment 6. The co-cultivation system according to any one of Embodiments 1 to 5, in which the feeding strain and/or the production strain are selected from one or more of bacteria, fungi or yeasts.

Embodiment 7. The co-cultivation system according to claims 1 to 6, wherein the metabolic pathway of the feeding strain and/or the production strain comprise an endogenous isoprenoid pathway.

Embodiment 8. A method for synthesizing terpenes using the co-cultivation system described in any one of Embodiments 1 to 7, comprising incubating the feeding strain and the production strain in the presence of an inducer and separating and purifying the terpene with organic phase or solid phase resins.

Embodiment 9. The method according to Embodiment 8, wherein based on the total inoculation volume of the feeding strain and the production strain, the content of the feeding strain is 0.25 % to 90%, preferably 0.5% to 75%; and/or the organic phase is selected from decane, undecane, dodecane, preferably decane; or the organic phase is selected from isopropyl decanoate, isopropyl laurate, isopropyl octanoate, isopropyl myristate; or solid phase resins is selected from Diaion HP20, Dowax 20, XAD7HP, HP-2MG, preferably Diaion HP20; and the organic phase is present at 5%-30% v/v of the culture, preferably 10%-20% v/v.

Embodiment 10. Use of the co-cultivation system according to any one of Embodiments 1 to 7 or the method according to Embodiment 8 or 9 in synthesizing terpenes.

From previous reports of terpene production in microorganisms, the plasmid pBbA5c-MevT(CO)-T1-MBIS(CO, ispA) was obtained from Addgene and the codon optimized terpene synthase was cloned into the pTRC-Hisa vector (Invitrogen). Both plasmids were transformed into *E. coli* BL21 (DE3). Transformed cells were grown until the OD at 600 nm reached 0.6 and then induced with 0.1 mM IPTG and incubated at 16°C or 30°C. 2% w/v glucose was added as a carbon source and 10% v/v decane was added as an organic phase to extract terpenes. This system produced reasonable levels of terpenes for soluble terpene synthase enzymes and lower levels for insoluble enzymes. The reason for these low terpene production levels may be the stress associated with the expression of heterologous enzymes. This barrier can be overcome by dividing or sharing the metabolic stress among different strains. Co-culture or two-strain metabolic engineering is emerging as an important area of research to reduce the number of heterologously expressed genes in a single strain. With this approach in mind, the present inventors investigated a method for dividing the associative terpene biosynthesis pathway between microbes and providing an example of a two-strain cultivation system.

The present inventors have found that the division of terpene biosynthetic pathways requires an intermediate that can be transferred between two strains and that does not accumulate in extractants such as decane. Isoprene units (IPP and DMAPP) (Niu et al., Frontiers in Microbiology, 2018, 9, 16232; DOI: 10.3389/fmicb.2018.01623) and terpene olefins (Zhou et al., Nature Biotechnology, 2015, 33, 377-383; DOI: 10.1038/nbt.3095) have been used as transfer intermediates between strains but the production efficiency is low. Bacteria and yeasts co-cultivation with terpene olefins as transfer intermediates resulted in the biosynthesis of 33 mg/L oxygenated taxanes, 20 mg/L ferruginol and 5 mg/L nootkatone (Zhou et al., Nature Biotechnology, 2015, 33, 377-383; DOI: 10.1038/nbt.3095). Co-culture of two strains with isoprene units as transfer intermediates (Figure 2A) produced 166 mg/L of pinene (Niu et al., Frontiers in Microbiology, 2018, 9, 16232; DOI: 10.3389/fmicb.2018.01623). A two-step culture system using mevalonic acid as an intermediate (Figure 2B) produced 150 mg/L sabinene in bacteria (Liu et al., Process Biochemistry, 2017, 62, 1-9; DOI: 10.1016/j.procbio.2017.07.021). The present invention investigates the terpene biosynthesis pathway, and there is currently no prior art that uses mevalonic acid as a transfer intermediate in a co-cultivation system. Mevalonic acid is a polar small molecule that diffuses easily through microbial membranes and does not partition into organic extractants such as decane.

During pinene biosynthesis, both strains must be grown in medium and the cells harvested then transferred to phosphate buffered saline to produce terpenes (Niu et al., Frontiers in Microbiology, 2018, 9, 16232; DOI: 10.3389/fmicb.2018.01623) (Figure 2A). Isoprene units are used as exchange metabolites that can cause stress in bacteria. During sabinene biosynthesis, mevalonic acid-producing bacteria must be grown in a medium; the resulting medium containing mevalonic acid is used with the growth medium of sabinene biosynthetic strains. To solve this problem, the present invention provides a simple co-cultivation system (Figure 3), in which one strain (the feeding strain) releases mevalonic acid into the growth medium and the other strain (production strain) uses mevalonic acid to synthesize terpenes. The method is easy to implement and has been demonstrated by the production of different terpenes such as taxadiene, α-bulnesene, valencene, α-guaiene, β-patchoulene and aristolochene (Figure 1).

The bacterial feeding strain and the bacterial production strain contain inbuild MEP pathway and part of engineered heterologous MVA pathway. The MEP pathway enables bacterial growth in minimal media without supplementation of isoprene intermediates. Furthermore, the engineered MVA pathway increases the flux for terpene biosynthesis.

The technical solutions of the present application will be exemplarily described below by way of examples.

The MVA pathway plasmid pBbA5c-MevT(CO)-T1-MBIS(CO,ispA) (pMVA) from Addgene (http://www.addgene.org/35152/; US7183089 and US736882; Tsuruta et al., PLoS One, 2009, 4, e4489; DOI: 10.1371/journal.pone.0004459; Peralta-Yahya, et al., Nature Communications, 2011, 2, 483; DOI: 10.1038/ncomms1494) was modified to work in a co-cultivation system. The pMVA plasmid is a medium copy number plasmid containing two modules, one for the production of mevalonic acid and the other for the production of farnesyl pyrophosphate (FPP). New plasmids pMevt and pMBIS were generated by cloning each module from pMVA. pMevt is a medium copy number plasmid containing a mevalonic acid production module. pMBIS is a medium copy number plasmid containing the FPP production module.

The pMevT and pMBIS plasmids in the present invention are derived from the Addgene pMVA plasmid, which is optimized for a single-strain system. The pMevT and pMIBS plasmids can be further optimized by modifying the plasmid copy number, promoter and RBS (ribosome binding site). Feeding and production strains can be further optimized by improving gene dosage, heterologous enzyme expression, efficiency, and host genome modifications.

### [Example 1.1] Taxadiene Biosynthesis

The codon-optimized genes for taxadiene synthase and geranylgeranyl pyrophosphate synthase were fused (Ajikumar, et al., Science, 2010, 330, 70-74; DOI: 10.1126/science.1191652) and cloned into pTRC-HisA to generate the plasmid pTRC-TXSGPPS.

### Single-strain system

A single-strain system for taxadiene production was obtained by transforming the *E. coli* BL21 (DE3) strain with pTRC-TXSGPPS and pMVA plasmids. Single colonies of transformed *E. coli* BL21 (DE3) were inoculated into 5 mL of LB (Luria-Bertani) medium and incubated at 37 °C for 12 hours to obtain overnight cultures. These overnight cultures were inoculated into 25 mL of TB (Terrific Broth) medium and incubated at 37 °C and 200 rpm. Once the OD value at 600 nm reached 0.6, the cultures were induced with 0.1 mM IPTG and incubated at 20 °C. 2% w/v glucose was added as a carbon source and 10% v/v decane was added as organic phase to extract terpenes.

### Two-strain co-cultivation system:

For the two-strain co-cultivation system, feeding strains were generated by transforming pMevt and pUC-19 plasmids into *E. coli* BL21 (DE3), and the taxadiene-producing strains were generated by transforming pMBIS and pTRC-TXSGPPS plasmids into *E. coli* BL21 (DE3). Colonies of feeding and production strains were independently inoculated into separate 5 mL of LB (Luria-Bertani) medium and incubated at 37 °C for 12 hours to obtain overnight cultures. Different ratios (50% to 12.5%) of overnight cultures of feeding and production strains were inoculated into 25 mL of TB (Terrific Broth) medium and incubated at 37 °C and 200 rpm. Once the OD value at 600 nm reached 0.6, the cultures were induced with 0.1 mM IPTG and incubated at 20 °C. 2% w/v glucose was added as a carbon source and 10% v/v decane was added as organic phase to extract terpenes.

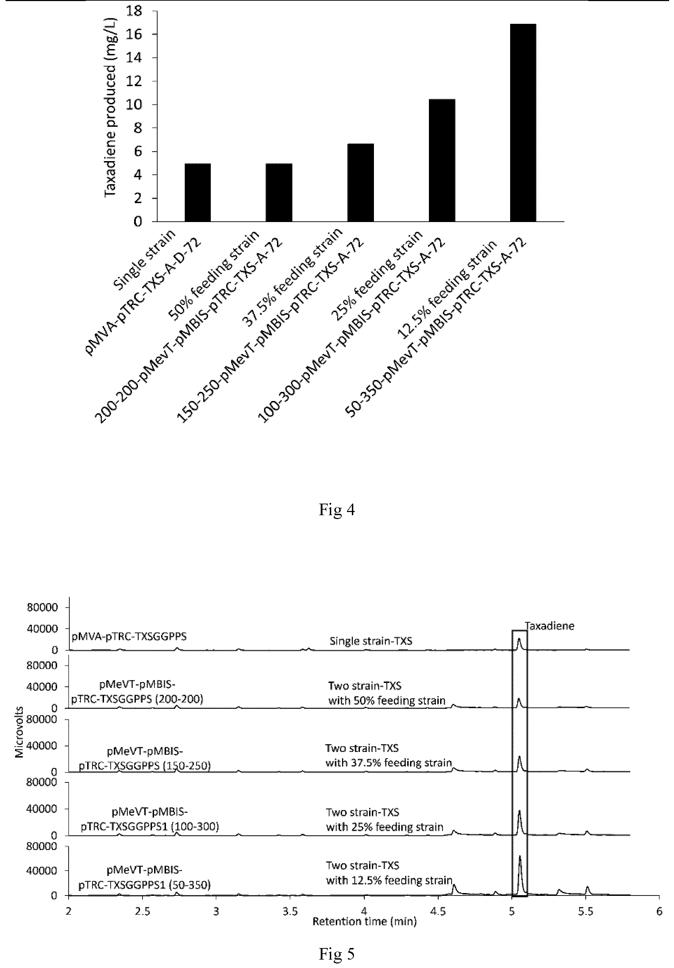

The results are shown in Figures 4 and 5. The results showed that the taxadiene yield reached nearly 6 mg/L in the single-strain system. For the two-strain co-cultivation system, the yield of taxadiene increased as the proportion of feeding strains decreased. In a two-strain co-cultivation system with 12.5% feeding strain in the initial inoculum, a maximum yield level of ~16 mg/L was observed, representing a nearly 3-fold increase in taxadiene yield.

### [Example 1.2] Taxadiene Biosynthesis

The codon-optimized genes for taxadiene synthase and geranylgeranyl pyrophosphate synthase were fused (Ajikumar, et al., Science, 2010, 330, 70-74; DOI: 10.1126/science.1191652) and cloned into pTRC-HisA to generate the plasmid pTRC-TXSGPPS.

### Single-strain system:

A single-strain system for taxadiene production was obtained by transforming the *E. coli* BL21 (DE3) strain with pTRC-TXSGPPS and pMVA plasmids. Single colonies of transformed *E. coli* BL21 (DE3) were inoculated into 5 mL of LB (Luria-Bertani) medium and incubated at 37 °C for 12 hours to obtain overnight cultures. These overnight cultures were inoculated into 25 mL of TB (Terrific Broth) medium and incubated at 37 °C and 200 rpm. Once the OD value at 600 nm reached 0.6, the cultures were induced with 0.1 mM IPTG and incubated at 20 °C. 2% w/v glucose was added as a carbon source and 10% v/v decane was added as organic phase to extract terpenes. Cultures were induced twice in 24-hour intervals with 0.1 mM IPTG and supplemented with 2% glucose and 2% tryptone. The decane phase was collated by centrifugation after 72 hours and analyzed by GC.

### Two-strain co-cultivation system:

For the two-strain co-cultivation system, feeding strains were generated by transforming pMevt and pUC-19 plasmids into *E. coli* BL21 (DE3), and the taxadiene-producing strains were generated by transforming pMBIS and pTRC-TXSGPPS plasmids into *E. coli* BL21 (DE3). Colonies of feeding and production strains were independently inoculated into separate 5 mL of LB (Luria-Bertani) medium and incubated at 37 °C for 12 hours to obtain overnight cultures. Different ratios (0.125% to 12.5%) of overnight cultures of feeding and production strains were inoculated into 25 mL of TB (Terrific Broth) medium and incubated at 37 °C and 200 rpm. Once the OD value at 600 nm reached 0.6, the cultures were induced with 0.1 mM IPTG and incubated at 20 °C. 2% w/v glucose was added as a carbon source and 10% v/v decane was added as organic phase to extract terpenes. Cultures were induced twice in 24-hour intervals with 0.1 mM IPTG and supplemented with 2% glucose and 2% tryptone. The decane phase was collated by centrifugation after 72 hours and analyzed by GC.

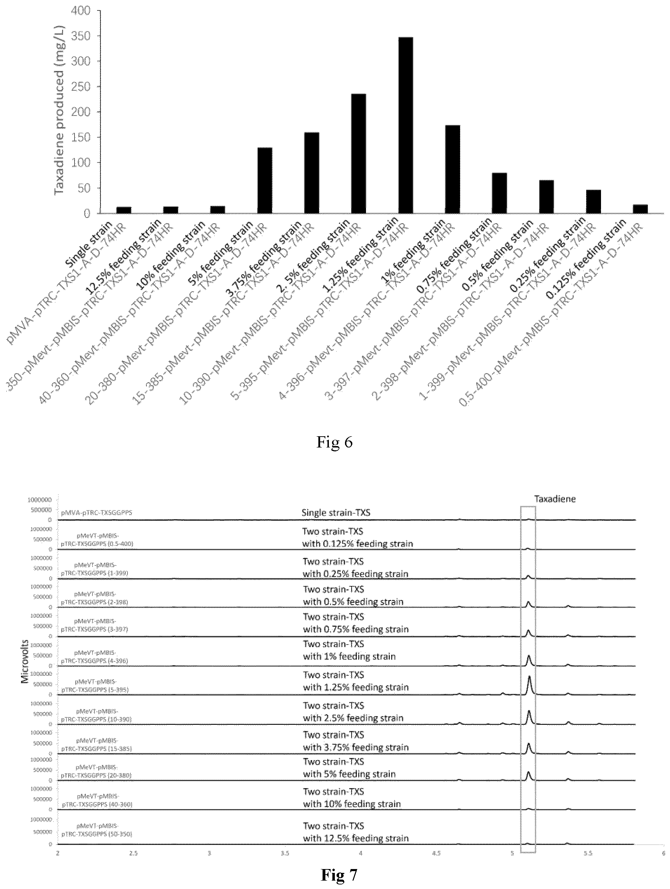

The results are shown in Figures 6 and 7. The results showed that the taxadiene yield reached nearly 13 mg/L in the single-strain system. For the two-strain co-cultivation system, the yield of taxadiene increased as the proportion of feeding strains decreased. In a two-strain co-cultivation system with 1.25% feeding strain in the initial inoculum, a maximum yield level of ~340 mg/L was observed, representing a nearly 25-fold increase in taxadiene yield.

### [Example 2] α-Bulnesene biosynthesis

The codon-optimized α-bulnesene synthase gene (NCBI - KF800046) was cloned into pTRC-HisA to generate plasmid pTRC-BS.

### Single-strain system:

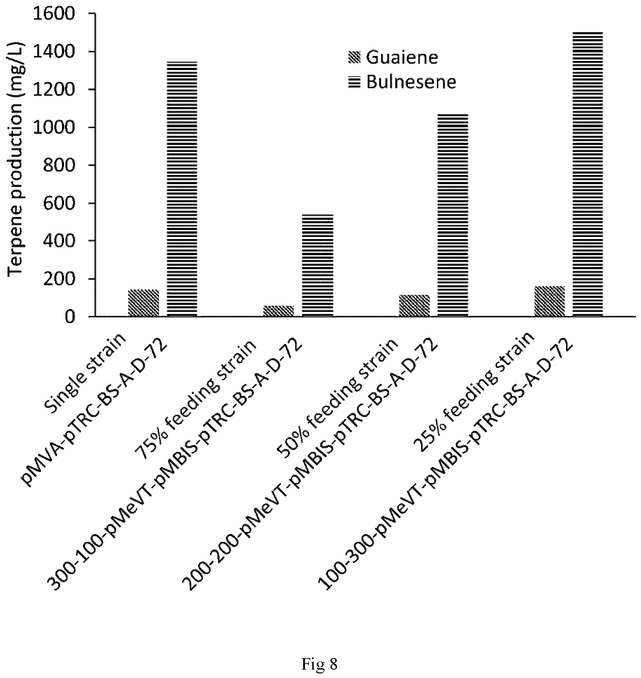

A single-strain system for α-bulnesene production was generated by transforming *E. coli* BL21 (DE3) with pMVA and pTRC-BS plasmids. Single colonies were inoculated into 5 mL of LB (Luria-Bertani) medium and incubated at 37 °C for 12 hours. These overnight cultures were inoculated into 25 mL of TB (Terrific Broth) medium and incubated at 37 °C and 200 rpm. Once the OD value at 600 nm reached 0.6, the cultures were induced with 0.1 mM IPTG and incubated at 30 °C. 2% w/v glucose was added as a carbon source and 10% v/v decane was added as organic phase to extract terpenes. The total terpene yield reached 1345 mg/L (Figure 8).

### Two-strain co-cultivation system:

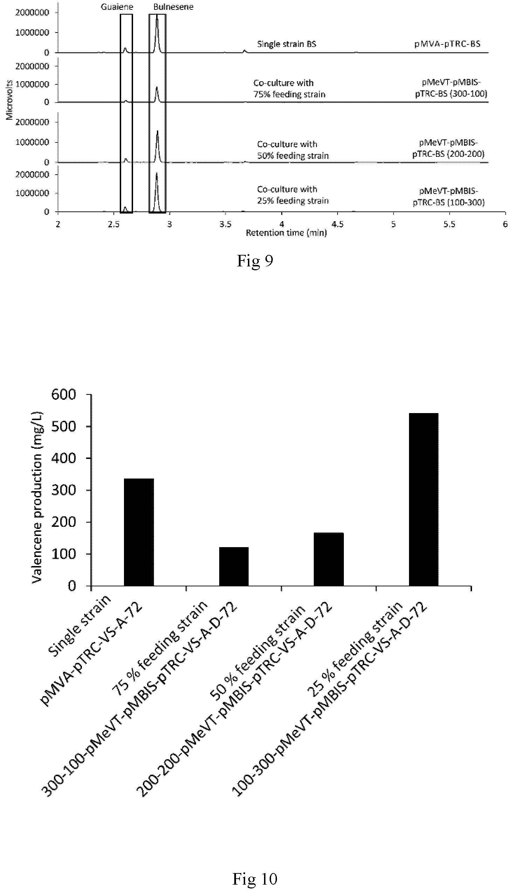

For the two-strain co-cultivation system, the feeding strains were generated by transforming the pMevt and pUC-19 plasmids into *E. coli* BL21 (DE3). Production strains for bulnesene synthesis were generated by transforming the pMBIS and pTRC-BS plasmids into *E. coli* BL21 (DE3). Colonies of feeding and production strains were inoculated into separate 5 mL LB (Luria-Bertani) medium and incubated at 37 °C for 12 hours to obtain overnight cultures. Different ratios (25% to 75%) of overnight cultures of feeding and production strains were inoculated into 25 mL of TB (Terrific Broth) medium and incubated at 37 °C and 200 rpm. Once the OD at 600 nm reached 0.6, cultures were induced with 0.1 mM IPTG and incubated at 30 °C. 2% w/v glucose was added as carbon source and 10% v/v decane was added as organic phase to extract terpenes. The two-strain co-cultivation system with 25% of the feeding strain produced 1500 mg/L of total terpenes, which is 10% higher than the single-strain system (Figure 9).

### [Example 3] Valencene Biosynthesis

The codon-optimized valencene synthase gene (from US9303252B2) was cloned into pTRC-HisA to generate plasmid pTRC-VS.

### Single-strain system:

A single-strain system for valencene biosynthesis was generated by transforming *E. coli* BL21 (DE3) with pMVA and pTRC-VS plasmids. Single colonies were inoculated into 5 mL of LB (Luria-Bertani) medium and incubated at 37 °C for 12 hours to obtain overnight cultures. These overnight cultures were inoculated into 25 mL of TB (Terrific Broth) medium and incubated at 37 °C and 200 rpm. Once the OD value at 600 nm reached 0.6, cultures were induced with 0.1 mM IPTG and incubated at 30 °C. 2% w/v glucose was added as a carbon source and 10% v/v decane was added as organic phase to extract terpenes. The total terpene production reached about 336 mg/L (Figure 10).

### Two-strain co-cultivation system:

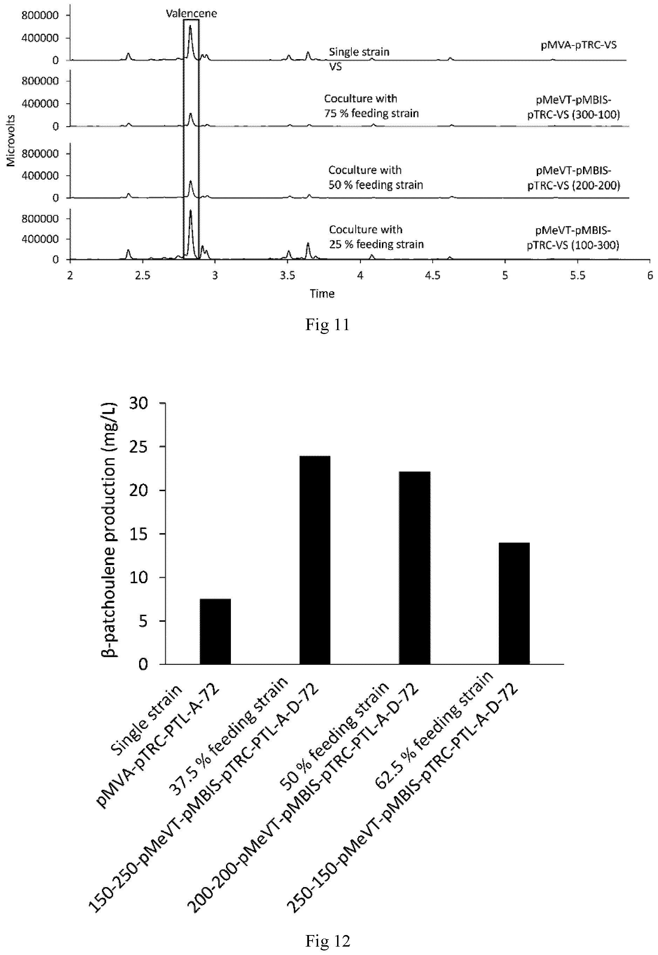

For the two-strain co-cultivation system, feeding strains were generated by transforming the pMevt and pUC-19 plasmids into *E. coli* BL21 (DE3). Production strains for valencene biosynthesis were generated by transforming the plasmids pMBIS and pTRC-VS into *E. coli* BL21 (DE3). Colonies of feeding and production strains were inoculated into separate 5 mL LB (Luria-Bertani) medium and incubated at 37 °C for 12 hours. Different ratios (25% to 75%) of overnight cultures of the feeding and production strains were inoculated into 25 mL of TB (Terrific Broth) medium and incubated at 37 °C and 200 rpm. Once the OD value at 600 nm reached 0.6, cultures were induced with 0.1 mM IPTG and incubated at 30 °C. 2% w/v of glucose was added as a carbon source and 10% v/v of decane was added to extract terpenes. The two-strain co-cultivation system with 25% feeding strains produced 540 mg/L of total terpenes, which is 35% higher than the single-strain system (Figure 11).

### [Example 4] β-Patchoulene biosynthesis

The codon-optimized β-patchoulene synthase gene (XM_044587644) was cloned into pTRC-HisA to generate plasmid pTRC-PCL.

### Single-strain system:

A single-strain system for β-patchoulene biosynthesis was generated by transforming *E*. *coli* BL21 (DE3) with pMVA and pTRC-PCL plasmids. Single colonies were inoculated into 5 mL of LB (Luria-Bertani) medium and incubated at 37 °C for 12 hours to obtain overnight cultures. These overnight cultures were inoculated into 25 mL of TB (Terrific Broth) medium and incubated at 37 °C and 200 rpm. Once the OD value at 600 nm reached 0.6, the cultures were induced with 0.1 mM IPTG and incubated at 16 °C. 2% w/v glucose was added as a carbon source and 10% v/v decane was added as organic phase to extract terpenes. The total terpene production reached about 7 mg/L (Figure 12).

### Two-strain co-cultivation system:

For the two-strain co-cultivation system, feeding strains were generated by transforming the pMevt and pUC-19 plasmids into *E. coli* BL21 (DE3). Production strains for β-patchoulene biosynthesis were generated by transforming the plasmids pMBIS and pTRC-PCL into *E. coli* BL21 (DE3). Colonies of feeding and production strains were inoculated into separate 5 mL LB (Luria-Bertani) medium and grown at 37 °C for 12 hours to obtain overnight cultures. Different ratios (37.5% to 62.5%) of overnight cultures of the feeding and production strains were inoculated into 25 mL of TB (Terrific Broth) medium and incubated at 37 °C and 200 rpm. Once the OD at 600 nm reached 0.6, cultures were induced with 0.1 mM IPTG and incubated at 16 °C. 2% w/v glucose was added as a carbon source and 10% v/v decane was added to extract terpenes. The two-strain co-cultivation system with 37.5% feeding strains produced 25 mg/L of total terpenes, which is three times that of the single-strain system (Figure 12).

### [Example 5] Biosynthesis of aristolochene

The codon-optimized aristolochene synthase gene (PDB-3M01_A) was cloned into pTRC-HisA to generate plasmid pTRC-TEAS.

### Single-strain system:

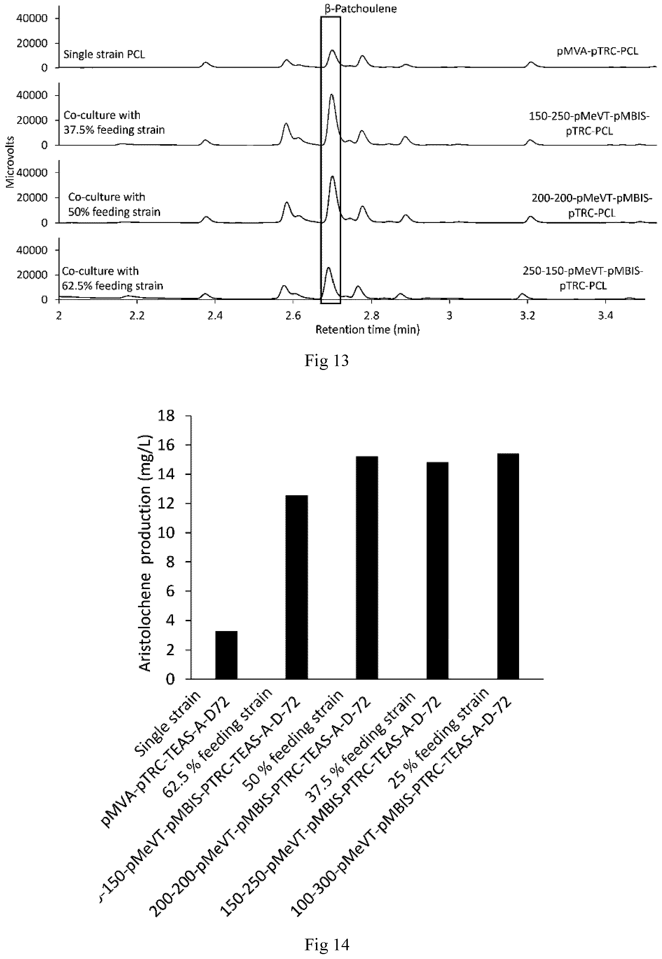

A single-strain system for aristolochene biosynthesis was generated by transforming E. *coli* BL21 (DE3) with pMVA and pTRC-TEAS plasmids. Single colonies were inoculated into 5 mL of LB (Luria-Bertani) medium and incubated at 37 °C for 12 hours to obtain overnight cultures. These overnight cultures were inoculated into 25 mL of TB medium and incubated at 37 °C and 200 rpm. Once the OD value at 600 nm reached 0.6, the cultures were induced with 0.1 mM IPTG and incubated at 30 °C. 2% w/v glucose was added as a carbon source and 10% v/v decane was added as organic phase to extract terpenes. The total terpene production reached about 3 mg/L (Figure 14).

### Two-strain co-cultivation system:

For the two-strain co-cultivation system, feeding strains were generated by transforming the pMevt and pUC-19 plasmids into *E. coli* BL21 (DE3). Production strains for β-aristolochene biosynthesis were generated by transforming the plasmids pMBIS and pTRC-TEAS into *E. coli* BL21 (DE3). Colonies of feeding and production strains were inoculated into separate 5 mL LB (Luria-Bertani) medium and incubated at 37 °C for 12 hours. Different ratios (25% to 62.5%) of the overnight feeding and production strains were inoculated into 25 mL of TB (Terrific Broth) medium and incubated at 37 °C and 200 rpm. Once the OD at 600 nm reached 0.6, cultures were induced with 0.1 mM IPTG and incubated at 30 °C. 2% w/v of glucose was added as a carbon source and 10% v/v of decane was added to extract terpenes. A two-strain co-cultivation system with 25% feeding strains produced 15 mg/L of total terpenes, which is five times that of a single-strain system (Figure 14).

While some exemplary embodiments of the present application have been illustrated and described, the present application is not limited to the disclosed embodiments. Rather, those of ordinary skill in the art will recognize that certain modifications and changes can be made to the described embodiments without departing from the spirit and scope of the application as described in the appended claims.

## Claims

1. A co-cultivation system for synthesizing terpenes, comprising at least one feeding strain and at least one production strain, wherein the feeding strain is used to provide mevalonic acid and the production strain is used to synthesize terpenes from the mevalonic acid.

2. The co-cultivation system according to claim 1, wherein the feeding strain contains a first plasmid, and the first plasmid contains a gene for synthesizing mevalonic acid and does not contain a gene for the synthesis of prenyl pyrophosphate; preferably, the feeding strain further contains a second plasmid, the second plasmid is a blank plasmid without gene insertion.

3. The co-cultivation system according to claim 1 or 2, wherein the production strain contains a third plasmid and a fourth plasmid, and the third plasmid contains genes for synthesizing farnesyl pyrophosphate and does not contain the gene for mevalonic acid synthesis, and the fourth plasmid contains the gene for the terpene synthase or fused gene of terpene synthase and geranylgeranyl pyrophosphate.

4. The co-cultivation system according to any one of claims 1 to 3, wherein the ratio of the feeding strain to the production strain is (0.25-99.75) : (99.75-0.25), preferably (1.25-75) : (98.75-25).

5. The co-cultivation system according to any one of claims 1 to 4, wherein the terpene is selected from the group of taxadiene, α-bulnesene, valencene, α-guaiene, β-patchoulene or aristolochene.

6. The co-cultivation system according to any one of claims 1 to 5, wherein the feeding strain and/or the production strain are selected from one or more of bacteria, fungi or yeasts.

7. The co-cultivation system according to any one of claims 1 to 6, wherein the metabolic pathway of the feeding strain and/or the production strain comprises an endogenous isoprenoid pathway.

8. A method for synthesizing terpenes using the co-cultivation system described in any one of claims 1 to 7, comprising incubating the feeding strain and the production strain in the presence of an inducer and an organic phase or solid phase resin for separating the terpene from the aqueous phase.

9. The method according to claim 8, wherein based on the total inoculation volume of the feeding strain and the production strain, the content of the feeding strain is 0.25% to 90%, preferably 0.5% to 75%;
and/or the organic phase is selected from decane, undecane, dodecane, isopropyl decanoate, isopropyl laurate, isopropyl octanoate, isopropyl myristate, preferably decane, and
the solid phase resin is selected from Diaion HP20, Dowax 20, XAD7HP, HP-2MG, preferably Diaion HP20; and the organic phase is present at 5% to 30% v/v of the culture, preferably 10% to 20% v/v.

10. The application of the co-cultivation system according to any one of claims 1 to 7 or the method according to any one of claims 8 and 9 in synthesizing terpenes.
